# EUROPÄISCHE PATENTSCHRIFT

(11) **EP 3 272 285 B1**
(45) Veröffentlichungstag und Bekanntmachung des Hinweises auf die Patenterteilung: **05.06.2019**
(21) Anmeldenummer: 17182465.9
(22) Anmeldetag: 20.07.2017
(51) Int. Cl.: A61B 5/15, A61B 5/151

(54) **VORRICHTUNG UND VERFAHREN ZUM MESSEN VON KENNGROESSEN ODER PARAMETERN EINER KOERPERFLUESSIGKEIT**
DEVICE AND METHOD FOR MEASURING VARIABLES OR PARAMETERS OF A BODILY FLUID
DISPOSITIF ET PROCÉDÉ DE MESURE DES CARACTÉRISTIQUES OU DES PARAMÈTRES D'UN FLUIDE CORPOREL

(30) Priorität: 20.07.2016 DE 102016113433
(43) Veröffentlichungstag der Anmeldung: 24.01.2018
(73) Patentinhaber: Techkon GmbH, 61462 Koenigstein im Taunus (DE)
(72) Erfinder: KRZYMINSKI, Ulrich, 61476 Kronberg im Taunus (DE)
(74) Vertreter: Hofmann, Andreas

(56) Entgegenhaltungen:
- WO-A1-2004/044571
- WO-A2-2005/107594
- US-A1- 2005 106 713
- US-A1- 2008 300 508

## Beschreibung

### Technisches Gebiet

Die vorliegende Erfindung betrifft eine Vorrichtung sowie ein Verfahren zum Messen mindestens einer Kenngröße oder mindestens eines Parameters mindestens einer Körperflüssigkeit.

### Stand der Technik

Konventionelle Systeme und Verfahren zum, insbesondere invasiven, Messen von Kenngrößen oder Parametern von Körperflüssigkeit(en) sind im Regelfall kompliziert sowie umständlich in der Handhabung und erfordern eine Vielzahl einzelner Zubehörteile, die die Benutzerin oder der Benutzer stets mit sich führen muss.

Der naheliegende Stand der Technik wird wiedergegeben durch die Druckschriften WO 2004/044571 A1, US2005/0106713 A1, US 2008/0300508 A1 und WO 2005/107594 A2.

### Darstellung der vorliegenden Erfindung: Aufgabe, Lösung, Vorteile; bester Weg zur Ausführung der vorliegenden Erfindung

Ausgehend von den vorstehend dargelegten Nachteilen sowie unter Würdigung des umrissenen Stands der Technik liegt der vorliegenden Erfindung die Aufgabe zugrunde, eine Vorrichtung sowie ein Verfahren der eingangs genannten Art so weiterzuentwickeln, dass die Körperflüssigkeit möglichst bedienerfreundlich und kompakt separiert sowie mindestens eine Kenngröße oder mindestens ein Parameter der Körperflüssigkeit auf möglichst einfache Weise bestimmt werden kann.

Erfindungsgemäß wird diese Aufgabe durch eine Vorrichtung mit den Merkmalen des Anspruchs 1 sowie durch ein Verfahren mit den Merkmalen des Anspruchs 10 gelöst. Vorteilhafte Ausgestaltungen und zweckmäßige Weiterbildungen der vorliegenden Erfindung sind in den jeweiligen Unteransprüchen gekennzeichnet.

Die vorliegende Erfindung betrifft also eine Vorrichtung gemäß des Anspruchs 1.

Mittels einer derartigen Vorrichtung kann die Körperflüssigkeit, zum Beispiel das Blut, bedienerfreundlich und kompakt im Pad separiert werden, insbesondere indem die Haut der Benutzerin oder des Benutzers mit dem Perforier-, Ritz- oder Stechmittel perforiert, geritzt oder gestochen wird, um auf diese invasive Weise an die Körperflüssigkeit, zum Beispiel an einen oder mehrere Bluttropfen, zu gelangen, und diese im Pad aufnehmen oder separieren zu können.

Unter Verwendung des Pads mit der aufgenommenen oder separierten Körperflüssigkeit kann dann die mindestens eine Kenngröße oder der mindestens eine Parameter, zum Beispiel der Blutzucker- oder Blutglucosewert, dieser Körperflüssigkeit auf einfache Weise bestimmt werden. Hierbei wird unter Blutzucker im Allgemeinen die Höhe des Glucoseanteils (Glucosespiegel) im Blut verstanden.

Um eine möglichst flache und kompakte Bauform der Vorrichtung zu gewährleisten, das heißt um die Dicke des kartenförmigen Trägers möglichst gering zu halten, kann das Perforier-, Ritz- oder Stechmittel, insbesondere zusammen mit dem Pad, in bevorzugter Weise zumindest partiell im Träger versenkbar sein.

Zu diesem Zweck kann (muss aber nicht) das Perforier-, Ritz- oder Stechmittel einen Federmechanismus aufweisen, durch den das Perforier-, Ritz- oder Stechmittel zumindest teilweise im Inneren des Trägers gelagert werden kann.

Erst bei Nutzung der Vorrichtung ragt dann das zumeist nadelartig ausgebildete Perforier-, Ritz- oder Stechmittel mit seiner Spitze aus der Ebene des Trägers heraus, um zum Beispiel eine Fingerkuppe der Benutzerin oder des Benutzers perforieren, ritzen oder stechen zu können und auf diese Weise an eine kleine Menge der Körperflüssigkeit zu gelangen.

Ebenfalls zu diesem Zweck kann (muss aber nicht) das Perforier-, Ritz- oder Stechmittel zusammen mit dem Pad in einer Senke oder Vertiefung des Trägers angeordnet sein. Hierdurch wird einerseits ebenfalls eine möglichst flache und kompakte Bauform der Vorrichtung gewährleistet, andererseits wird hierdurch ermöglicht, dass die Benutzerin bzw. der Benutzer ihren bzw. seinen Finger intuitiv in diese Senke oder Vertiefung legt, um den Messvorgang zu initiieren.

Gemäß der vorliegenden Erfindung ist das Pad zweiteilig ausgebildet, nämlich
- in Form eines die Körperflüssigkeit separierenden Moduls und
- in Form eines bezüglich der Körperflüssigkeit reaktiven Moduls.

Diese beiden Module können einander, insbesondere räumlich, zugeordnet sein, insbesondere in Kontakt miteinander stehen. Das separierende Modul kann sich unmittelbar an das Perforier-, Ritz- oder Stechmittel anschließen und dient zum Separieren und/oder Aufnehmen der vom Perforier-, Ritz- oder Stechmittel kommenden Körperflüssigkeit, die sodann an das reaktive Modul weitergegeben oder weitergeleitet werden kann.

Gemäß der vorliegenden Erfindung ist das Perforier-, Ritz- oder Stechmittel und das reaktive Modul voneinander beabstandet, also an verschiedenen Stellen angeordnet, nämlich indem das reaktive Modul der zweiten Oberfläche zugeordnet ist, das heißt durch Drehen des Trägers (zum Beispiel vor dem Drehen: erste Oberfläche oben, zweite Oberfläche unten; nach dem Drehen: zweite Oberfläche oben, erste Oberfläche unten) hat die Benutzerin oder Benutzer freien Blick auf das reaktive Modul.

Sodann kann die durch das reaktive Modul bestimmbare Kenngröße und/oder der durch das reaktive Modul bestimmbare Parameter durch mindestens ein zum reaktiven Modul in Beziehung setzbares Auswertemittel auswertbar sein, das heißt die Benutzerin oder der Benutzer kann zum Beispiel in Erfahrung bringen, ob und inwieweit sich der Blutzucker- oder Blutglucosewert nach einer Mahlzeit innerhalb vorgegebener Toleranzschwellen befindet oder nicht.

Hierzu sind im reaktiven Modul verschiedene chemische Stoffe oder Reagenzien eingelagert, die bei Kontakt mit der Körperflüssigkeit reagieren und auf dem reaktiven Modul einen entsprechenden Farbumschlag hervorrufen.

Hierbei kann das Auswertemittel durch auf die Kenngröße oder den Parameter bezogene Referenz- oder Vergleichsdaten gegeben sein, die auf der zweiten Oberfläche vorgesehen, zum Beispiel aufgedruckt, sind. Bei diesen Referenz- oder Vergleichsdaten kann es sich zum Beispiel um Farbfelder handeln, die durch die Benutzerin oder den Benutzer visuell mit einer Verfärbung des reaktiven Moduls abgeglichen werden kann.

Hierzu kann das reaktive Modul von verschiedenen Farbfeldern umgeben sein, wobei jede Farbe einem bestimmten Kenngrößen- oder Parameter-Bereich entspricht. Durch optisches Abgleichen der Farbe des reaktiven Moduls mit der Farbe des passenden Farbfelds kann die Benutzerin oder der Benutzer so den in seinem Körper derzeit gegebenen Kenngrößen- oder Parameter-Bereich in Erfahrung bringen.

Die Auswertung des Farbumschlags im reaktiven Modul kann also rein optisch oder visuell anhand der auf dem Träger abgedruckten Farbfelder oder Farbtabelle (sogenannte ColourChart) erfolgen. Allerdings kann die Genauigkeit dieser Tests durch Umgebungslicht, durch die individuelle Sehkraft der Benutzerin oder des Benutzers und/oder durch die eingeschränkte Druckqualität der Farbfelder oder der Farbtabelle eingeschränkt sein.

Alternativ oder ergänzend, zum Beispiel zum Präzisieren oder Spezifizieren der mittels der Farbfelder ermittelten Kenngrößen- oder Parameter-Bereiche, kann das Auswertemittel durch mindestens eine auf die Kenngröße oder den Parameter bezogene externe Anwendung, zum Beispiel durch ein Datenverarbeitungsprogramm oder durch eine App(likation), oder externe Einrichtung, zum Beispiel ein mobiles Endgerät, gegeben sein.

Hier liegt der Vorteil in der Genauigkeit im Vergleich zum optischen oder visuellen Verfahren mittels der Farbfelder. Besonders vorteilhaft ist hier zusätzlich, dass das Analyse-Ergebnis verwechslungssicher digital erfasst und zur medizinischen Ferndiagnose übertragen werden kann. Dadurch liefert diese Technologie einen wesentlichen Beitrag für Telemedizin-/ eHealth-Anwendungen.

Das Datenverarbeitungsprogramm kann beispielsweise auf einem Tablet-Computer oder die App(likation) auf einem Smartphone installiert sein. Mittels der Kamera des mobilen Endgeräts kann die Verfärbung des reaktiven Pads durch das mobile Endgerät erfasst und sodann mittels des Datenverarbeitungsprogramms oder der App ausgewertet werden, so dass der Kenngrößen- oder Parameter-Wert, wie etwa der Blutzucker- oder Blutglucosewert, auf dem Display des mobilen Endgeräts angezeigt und die Benutzerin bzw. der Benutzer hieraus ihre bzw. seine Schlussfolgerungen ziehen kann.

Hierbei wird der Farbumschlag des reaktiven Moduls durch das Datenverarbeitungsprogramm oder durch die App farbverbindlich erfasst und den entsprechenden Referenz-Farbwerten der Analyse-Parameter zugeordnet. Das Ergebnis sind objektive, digitale Analysedaten, die über das mobile Endgerät direkt an ein Labor geschickt oder als Trendverlauf gespeichert werden können.

In einer besonders zweckmäßigen Ausgestaltungsform der vorliegenden Erfindung kann das Perforier-, Ritz-oder Stechmittel zusammen mit dem Pad durch eine, insbesondere wiederverschließbare, Abdeckung, zum Beispiel in Form einer Schutzfolie, abgedeckt sein.

Dies dient zum einem dem Schutz, zum Beispiel vor Verschmutzung, des sich unter der Abdeckung befindlichen Perforier-, Ritz- oder Stechmittels zusammen mit dem Pad; zum anderen wird durch diese Abdeckung auch die Benutzerin oder der Benutzer davor bewahrt, sich unbeabsichtigt mit dem Perforier-, Ritz- oder Stechmittel zu verletzen.

Zur Entnahme der Körperflüssigkeit wird die Abdeckung nach oben abgehoben oder weggezogen und auf diese Weise das Perforier-, Ritz- oder Stechmittel zusammen mit dem Pad freigelegt, wobei ein das Perforier-, Ritz- oder Stechmittel nicht abdeckender Abschnitt der Abdeckung mit der ersten Oberfläche des Trägers verbunden bleibt.

Nach Entnahme der Körperflüssigkeit kann das Perforier-, Ritz- oder Stechmittel zusammen mit dem Pad wieder durch die Abdeckung abgedeckt, also wieder verschlossen werden, zum Beispiel bevor der Träger umgedreht wird (, wie oben beschrieben).

In bevorzugter Weise kann die Vorrichtung aus einer, insbesondere mit einer Öffnungsperforation versehenen und/oder im Wesentlichen transparenten und/oder, zum Beispiel mit einer Gebrauchsanleitung, bedruckten, Schutzhülle oder Verpackung entnehmbar und nach Gebrauch, insbesondere nach Einmal-Gebrauch, in dieser Schutzhülle oder Verpackung entsorgbar sein.

Um das Anwendungsspektrum der Vorrichtung gemäß der vorliegenden Erfindung erweitern zu können und/oder um die gemessenen Kenngrößen- oder Parameterwerte in Beziehung zu anderen Körper-Kenngrößen oder -Parametern der Benutzerin oder des Benutzers setzen zu können, können/kann an oder auf der Vorrichtung, insbesondere an oder auf dem Träger, zum Beispiel an oder auf der zweiten Oberfläche des Trägers,
- mindestens ein Id(entitäts)modul,
- mindestens ein Temperaturerkennungsmodul,
- mindestens ein Speichermodul, insbesondere in Form mindestens einer Chipeinheit, und/oder
- mindestens ein Datenübertragungsmodul
vorgesehen sein.

Anstelle Blut können mit der Vorrichtung auch andere Körperflüssigkeiten, wie etwa Fruchtwasser, Speichel und/oder Urin erfasst und für die medizinische Diagnostik ausgewertet werden.

Die vorliegende Erfindung betrifft des Weiteren ein Verfahren zum, insbesondere invasiven, Messen mindestens einer Kenngröße oder mindestens eines Parameters mindestens einer Körperflüssigkeit mittels einer Vorrichtung gemäß der vorstehend dargelegten Art, wobei die durch das Pad, insbesondere durch ein reaktives Modul des Pads, bestimmbare Kenngröße und/oder Parameter durch mindestens ein Auswertemittel auswertbar ist.

### Kurze Beschreibung der Zeichnungen

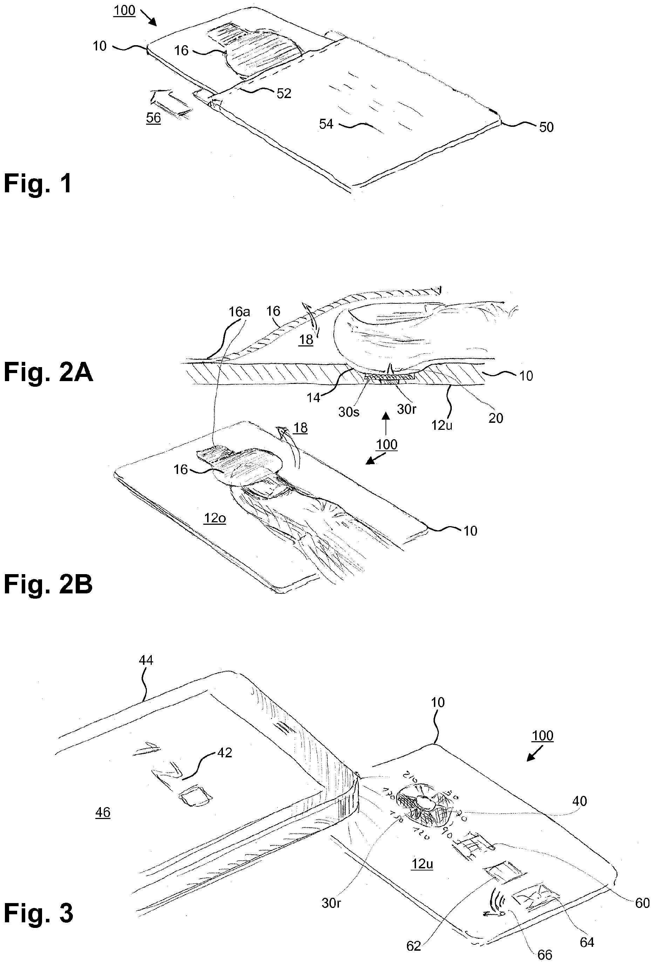

Wie bereits vorstehend erörtert, gibt es verschiedene Möglichkeiten, die Lehre der vorliegenden Erfindung in vorteilhafter Weise auszugestalten und weiterzubilden. Hierzu wird einerseits auf die dem Anspruch 1 nachgeordneten Ansprüche verwiesen, andererseits werden weitere Ausgestaltungen, Merkmale und Vorteile der vorliegenden Erfindung nachstehend unter anderem anhand des durch Fig. 1 bis Fig. 3 veranschaulichten Ausführungsbeispiels näher erläutert.

Es zeigt:
- Fig. 1: eine perspektivische Darstellung eines in einer sterilen Schutzhülle oder sterilen Verpackung aufgenommenen Ausführungsbeispiels einer Vorrichtung gemäß der vorliegenden Erfindung, die nach dem Verfahren gemäß der vorliegenden Erfindung zu benutzen ist;
- Fig. 2A: eine seitliche, zum Teil geschnittene Darstellung der Vorrichtung aus Fig. 1, die durch Auflegen und Stechen eines Fingers gerade benutzt wird;
- Fig. 2B: eine perspektivische Darstellung der gerade benutzten Vorrichtung aus Fig. 2A; und
- Fig. 3: eine perspektivische Darstellung der Möglichkeiten der Auswertung der mittels der Vorrichtung aus Fig. 1 bis Fig. 2B bestimmten Kenngrößen oder Parameter der Körperflüssigkeit.

Gleiche, ähnliche oder übereinstimmende Ausgestaltungen, Elemente oder Merkmale sind in Fig. 1 bis Fig. 3 mit denselben Bezugszeichen versehen; es wird auf eine wiederholte Beschreibung dieser Ausgestaltungen, Elemente oder Merkmale verzichtet.

### Bester Weg zur Ausführung der vorliegenden Erfindung

Die anhand Fig. 1 bis Fig. 3 veranschaulichte Einweg-Vorrichtung 100 dient zum Separieren und Aufnehmen einer Körperflüssigkeit, nämlich von Blut, sowie zum Bestimmen einer Kenngröße oder eines Parameters, nämlich des Blutzucker- oder Blutglucosewerts, des in der Vorrichtung 100 separierten und aufgenommenen Bluts. Hierbei wird unter Blutzucker die Höhe des Glucoseanteils (Glucosespiegel) im Blut verstanden.

Wie aus Fig. 1 hervorgeht, wird die Vorrichtung 100 aus einer mit einer Öffnungsperforation 52 versehenen, im Wesentlichen transparenten und mit einer Gebrauchsanweisung 54 bedruckten sterilen Verpackung 50 entnommen und kann nach einmaligem Gebrauch auch wieder in dieser Schutzhülle 50 entsorgt werden.

Die Vorrichtung 100 weist einen kartenförmigen Träger 10 mit einer ersten Oberfläche 12o und mit einer von der ersten Oberfläche 12o abgewandten zweiten Oberfläche 12u auf. Der Träger 10 kann, zumindest in etwa, die Größe einer Bankkarte, einer Kreditkarte oder einer SmartCard aufweisen.

Mittels der Vorrichtung 100 kann das Blut bedienerfreundlich und kompakt in einem Pad 30s, 30r separiert werden, indem die Haut an einer Fingerkuppe der Benutzerin oder des Benutzers mit einem an der ersten Oberfläche 12o des Trägers 10 vorgesehenen, aus der flächigen Erstreckung des Trägers 10 emporragenden nadelförmigen Perforier-, Ritz- oder Stechmittel 20 perforiert, geritzt oder gestochen wird, um auf diese invasive Weise an einen oder mehrere Bluttropfen zu gelangen.

Diese(r) Bluttropfen fließt entlang der Nadel 20 in Richtung des der Nadel 20 zugeordneten Pads 30s, 30r und wird in diesem aufgenommen oder separiert, so dass im Anschluss daran unter Verwendung des Pads 30s, 30r mit der aufgenommenen oder separierten Körperflüssigkeit der Blutzucker- oder Blutglucosewert bestimmt werden kann (vgl. Fig. 3).

Um eine möglichst flache und kompakte Bauform der Vorrichtung 100 zu gewährleisten, das heißt um die Dicke des kartenförmigen Trägers 10 möglichst gering zu halten, ist das Perforier-, Ritz- oder Stechmittel 20 zusammen mit dem Pad 30s, 30r zumindest partiell im Träger versenkt.

Zu diesem Zweck kann das Perforier-, Ritz- oder Stechmittel 20 einen Federmechanismus aufweisen, durch den das Perforier-, Ritz- oder Stechmittel 20 zumindest teilweise im Inneren des Trägers 10 gelagert werden kann.

Erst bei Nutzung der Vorrichtung 100 ragt dann das zumeist nadelartig ausgebildete Perforier-, Ritz- oder Stechmittel 20 mit seiner Spitze aus der Ebene des Trägers 10 heraus.

Ebenfalls zu diesem Zweck ist das Perforier-, Ritz- oder Stechmittel 20 zusammen mit dem Pad 30s, 30r in einer Senke oder Vertiefung 14 des Trägers 10 angeordnet. Hierdurch wird zum einen ebenfalls eine möglichst flache und kompakte Bauform der Vorrichtung 100 gewährleistet, zum anderen wird hierdurch ermöglicht, dass die Benutzerin bzw. der Benutzer ihren bzw. seinen Finger intuitiv in diese Senke oder Vertiefung 14 legt, um den Messvorgang zu initiieren.

Wie Fig. 2A entnehmbar, ist das Pad 30s, 30r zweiteilig ausgebildet, nämlich
- in Form eines das Blut separierenden Moduls 30s und
- in Form eines bezüglich des Bluts reaktiven Moduls 30r.

Diese beiden Module 30s, 30r sind einander räumlich benachbart zugeordnet und stehen in Kontakt miteinander. Das separierende Modul 30s schließt sich unmittelbar an das Perforier-, Ritz- oder Stechmittel 20 an und dient zum Separieren und Aufnehmen des mittels des Perforier-, Ritz- oder Stechmittels 20 aus der Fingerkuppe der Benutzerin oder des Benutzers entnommenen Bluts, das sodann an das reaktive Modul 30r weitergegeben oder weitergeleitet wird.

Gemäß der vorliegenden Erfindung sind das Perforier-, Ritz- oder Stechmittel 20 und das reaktive Modul 30r beabstandet voneinander, also an verschiedenen Stellen angeordnet, nämlich das Perforier-, Ritz-, oder Stechmittel 20 auf der ersten Oberfläche 12o und das reaktive Modul 30r auf der zweiten Oberfläche 12u.

Gemäß einer bevorzugten Ausführungsform der vorliegenden Erfindung kann bei der optischen oder visuellen Messung das Blut im separierenden Modul 30s über mindestens eine Kapillare zum auf der zweiten Oberfläche 12u des Trägers 10 sichtbaren reaktiven Modul 30r gezogen werden. In diesem als Testfeld ausgebildeten reaktiven Modul 30r sind verschiedene chemische Stoffe oder Reagenzien eingelagert, die mit dem Blut reagieren und eine Farbänderung des Testfelds zur Folge haben.

Das reaktive Modul 30r ist also der zweiten Oberfläche 12u des Trägers 10 zugeordnet, das heißt durch Drehen des Trägers (vgl. Fig. 2A vor dem Drehen: erste Oberfläche 12o oben, zweite Oberfläche 12u unten; Fig. 2B nach dem Drehen: zweite Oberfläche 12u oben, erste Oberfläche 12o unten) hat die Benutzerin oder Benutzer freien Blick auf das reaktive Modul 30r (vgl. Fig. 2B).

Sodann kann der durch das reaktive Modul 30r bestimmbare Blutzucker- oder Blutglucosewert durch ein zum reaktiven Modul 30r in Beziehung setzbares Auswertemittel 40 auswertbar sein, das heißt die Benutzerin oder der Benutzer kann zum Beispiel in Erfahrung bringen, ob und inwieweit sich der Blutzucker-oder Blutglucosewert nach einer Mahlzeit innerhalb vorgegebener Toleranzschwellen befindet oder nicht.

Hierbei ist das Auswertemittel 40 durch auf den Blutzucker- oder Blutglucosewert bezogene Referenz- oder Vergleichsdaten gegeben, die auf der zweiten Oberfläche 12u aufgedruckt sind. Bei diesen Referenz- oder Vergleichsdaten handelt es sich um Farbfelder 40, die durch die Benutzerin oder den Benutzer optisch oder visuell mit einer Verfärbung des reaktiven Moduls 30r abgeglichen werden können.

So ist aus der Beschriftung der jeweiligen Farbfelder 40 in der exemplarischen Darstellung der Fig. 3 ersichtlich, dass die jeweiligen Farbfelder 40 Blutzucker- oder Blutglucosewerten von 50 mg/dl, von 70 mg/dl, von 90 mg/dl, von 120 mg/dl, von 150 mg/dl, von 170 mg/dl und von 210 mg/dl entsprechen (bei einem gesunden Menschen betragen die Normalwerte nüchtern etwa 70 mg/dl nis 99 mg/dl und nach einer kohlenhydratreichen Mahlzeit maximal bis zu 160 mg/dl und nach zwei Stunden unter 140 mg/dl).

Um aus der Dauer und aus der Stärke der Farbänderung des als Testfeld ausgebildeten reaktiven Moduls 30r die Änderung des Blutzucker- oder Blutglucosewerts zu bestimmen, ist das reaktive Modul von den verschiedenen Farbfeldern 40 umgeben (vgl. Fig. 3), wobei jede Farbe einem bestimmten Bereich des Blutzucker- oder Blutglucosegehalts entspricht.

Durch optisches oder visuelles Abgleichen der Farbe des reaktiven Moduls 30r mit der passenden Farbe der Farbfelder 40 kann die Benutzerin bzw. der Benutzer so den in ihrem bzw. seinem Körper derzeit gegebenen Blutzucker- oder Blutglucosegehalt in Erfahrung bringen.

Alternativ oder ergänzend, zum Beispiel zum Präzisieren oder Spezifizieren des mittels der Farbfelder 40 ermittelten Blutzucker- oder Blutglucosebereichs, kann das Auswertemittel durch mindestens eine auf dem Blutzucker- oder Blutglucosewert bezogene externe Anwendung 42, zum Beispiel durch ein Datenverarbeitungsprogramm oder durch eine App(likation), und/oder externe Einrichtung 44, zum Beispiel ein mobiles Endgerät, gegeben sein.

Hier liegt der Vorteil in der Genauigkeit im Vergleich zum optischen oder visuellen Verfahren mittels der Farbfelder 40. Besonders vorteilhaft ist hier zusätzlich, dass das Analyse-Ergebnis verwechslungssicher digital erfasst und zur medizinischen Ferndiagnose übertragen werden kann. Dadurch liefert diese Technologie einen wesentlichen Beitrag für Telemedizin-/ eHealth-Anwendungen.

Das Datenverarbeitungsprogramm ist beispielsweise auf einem Tablet-Computer oder die App(likation) auf einem Smartphone installiert. Mittels der Kamera des mobilen Endgeräts 44 kann die Verfärbung des reaktiven Pads 30r durch das mobile Endgerät 44 erfasst und sodann mittels des Datenverarbeitungsprogramms oder der App ausgewertet werden, so dass der Blutzucker- oder Blutglucosewert auf dem Display des mobilen Endgeräts 44 angezeigt und die Benutzerin bzw. der Benutzer hieraus ihre bzw. seine Schlussfolgerungen ziehen kann.

Wie aus Fig. 1, Fig. 2A und Fig. 2B ersichtlich, ist das Perforier-, Ritz- oder Stechmittel 20 zusammen mit dem Pad 30s, 30r durch eine wiederverschließbare Abdeckung 16 in Form einer Schutzfolie abgedeckt. Diese Schutzfolie dient einerseits dem Schutz, zum Beispiel vor Verschmutzung, des sich unter der Abdeckung 16 befindlichen Perforier-, Ritz- oder Stechmittels 20 zusammen mit dem Pad 30s, 30r; andererseits wird durch diese Abdeckung 16 auch die Benutzerin oder der Benutzer davor bewahrt, sich unbeabsichtigt mit dem Perforier-, Ritz- oder Stechmittel 20 zu verletzen.

Zur Entnahme des Bluts wird die Abdeckung 16 in Richtung 18, also nach oben abgehoben und auf diese Weise das Perforier-, Ritz- oder Stechmittel 20 zusammen mit dem Pad 30s, 30r freigelegt, wobei ein das Perforier-, Ritz- oder Stechmittel 20 nicht abdeckender Abschnitt 16a der Abdeckung 16 mit der ersten Oberfläche 12o des Trägers 10 verbunden bleibt.

Nach Entnahme des Bluts wird das Perforier-, Ritz- oder Stechmittel 20 zusammen mit dem Pad 30s, 30r wieder durch die Abdeckung 16 abgedeckt, also wieder verschlossen, zum Beispiel bevor der Träger 10 umgedreht wird (, wie oben beschrieben).

Um das Anwendungsspektrum der Vorrichtung 100 erweitern zu können und/oder um die gemessenen Blutzucker- oder Blutglucosewerte in Beziehung zu anderen Körper-Parametern der Benutzerin oder des Benutzers setzen zu können, sind an oder auf der zweiten Oberfläche 12u des Trägers 10
- ein Id(entitäts)modul 60,
- ein Temperaturerkennungsmodul 62,
- ein Speichermodul 64 in Form einer Chipeinheit, insbesondere zum Abspeichern der Daten, und/oder
- ein Datenübertragungsmodul 66, insbesondere zum funklosen Übertragen der Daten,
vorgesehen.

Anstelle des oder zusätzlich zum Blutzucker- oder Blutglucosewert können auch andere Fluide, insbesondere weitere Körperflüssigkeiten, wie etwa das Fruchtwasser, der Speichel und/oder der Urin, gemessen und für die medizinische Diagnostik ausgewertet werden.

### Liste der Bezugszeichen

- 100: Vorrichtung, insbesondere Einweg-Vorrichtung
- 10: Träger, insbesondere kartenförmiger Träger
- 12o: erste Oberfläche des Trägers 10
- 12u: zweite Oberfläche des Trägers 10
- 14: Senke oder Vertiefung im Träger 10
- 16: Abdeckung, insbesondere wiederverschließbare Abdeckung, zum Beispiel Schutzfolie
- 16a: fest mit der ersten Oberfläche 12o verbundener Abschnitt der Abdeckung 16
- 18: Richtung der Bewegung der Abdeckung 16
- 20: Perforier-, Ritz- oder Stechmittel, insbesondere Nadel
- 30r: bezüglich der Körperflüssigkeit reaktives Modul, insbesondere Testfeld
- 30s: die Körperflüssigkeit separierendes Modul
- 40: Referenz- oder Vergleichsdaten als Auswertemittel, insbesondere Farbtabelle (ColourChart) oder verschiedene Farbfelder
- 42: Datenverarbeitungsprogramm oder App(likation) als Auswertemittel
- 44: externe Einrichtung, insbesondere mobiles Endgerät, zum Beispiel Smartphone oder Tablet-Computer
- 46: Anzeigeeinheit oder Display der externen Einrichtung 44
- 50: Schutzhülle oder Verpackung, insbesondere sterile Schutzhülle oder sterile Verpackung
- 52: Öffnungsperforation der Schutzhülle oder Verpackung 50
- 54: Gebrauchsanleitung auf Schutzhülle oder Verpackung 50
- 56: Richtung der Entnahme der Karte 10 aus Schutzhülle oder Verpackung 50
- 60: Id(entitäts)modul
- 62: Temperaturerkennungsmodul
- 64: Speichermodul, insbesondere Chipeinheit
- 66: Datenübertragungsmodul

## Patentansprüche

1. Vorrichtung (100), insbesondere Einweg-Vorrichtung, zum Messen mindestens einer Kenngröße oder mindestens eines Parameters mindestens einer Körperflüssigkeit, aufweisend
- mindestens einen kartenförmigen Träger (10) mit einer ersten Oberfläche (12o) und mit einer von der ersten Oberfläche (12o) abgewandten zweiten Oberfläche (12u),
- mindestens ein, insbesondere nadelförmiges, Perforier-, Ritz- oder Stechmittel (20) auf der ersten Oberfläche (12o) und
- mindestens ein dem Perforier-, Ritz- oder Stechmittel (20) zugeordnetes Pad (30s, 30r), mittels dessen die Körperflüssigkeit separierbar und/oder aufnehmbar sowie die mindestens eine Kenngröße oder der mindestens eine Parameter bestimmbar ist,
**dadurch gekennzeichnet,**
- **dass** das Pad (30s, 30r) zweiteilig ausgebildet ist, nämlich
-- in Form eines die Körperflüssigkeit separierenden, dem Perforier-, Ritz- oder Stechmittel (20) zugeordneten Moduls (30s) und
-- in Form eines bezüglich der Körperflüssigkeit reaktiven, der zweiten Oberfläche (12u) zugeordneten Moduls (30r),
- **dass** die durch das reaktive Modul (30r) bestimmbare Kenngröße und/oder der durch das reaktive Modul (30r) bestimmbare Parameter durch mindestens ein zum reaktiven Modul (30r) in Beziehung setzbares Auswertemittel (40; 42) auswertbar ist, wobei das Auswertemittel (40; 42)
-- durch auf die Kenngröße oder den Parameter bezogene Referenz- oder Vergleichsdaten, die auf der zweiten Oberfläche (12u) vorgesehen, zum Beispiel aufgedruckt, sind, und/oder
-- durch mindestens eine auf die Kenngröße oder den Parameter bezogene externe Anwendung oder externe Einrichtung
gegeben ist.

2. Vorrichtung gemäß Anspruch 1, wobei das Perforier-, Ritz- oder Stechmittel (20), insbesondere zusammen mit dem Pad (30s, 30r), zumindest partiell im Träger (10) versenkbar ist.

3. Vorrichtung gemäß Anspruch 1 oder 2, wobei das Perforier-, Ritz- oder Stechmittel (20) einen Federmechanismus, insbesondere zum zumindest partiellen Versenken des Perforier-, Ritz- oder Stechmittels (20) im Träger (10), aufweist.

4. Vorrichtung gemäß mindestens einem der Ansprüche 1 bis 3, wobei das Perforier-, Ritz- oder Stechmittel (20) zusammen mit dem Pad (30s, 30r) in einer Senke oder Vertiefung (14) des Trägers (10) angeordnet sind.

5. Vorrichtung gemäß mindestens einem der Ansprüche 1 bis 4, wobei das Auswertemittel (42) ein Datenverarbeitungsprogramm oder eine Applikation oder eine App ist.

6. Vorrichtung gemäß mindestens einem der Ansprüche 1 bis 5, wobei das das Perforier-, Ritz- oder Stechmittel (20) zusammen mit dem Pad (30s, 30r) durch eine, insbesondere wiederverschließbare, Abdeckung (16), zum Beispiel in Form einer Schutzfolie, abgedeckt ist.

7. Vorrichtung gemäß mindestens einem der Ansprüche 1 bis 6, wobei die Vorrichtung (100) aus einer, insbesondere mit einer Öffnungsperforation (52) versehenen und/oder im Wesentlichen transparenten und/oder, zum Beispiel mit einer Gebrauchsanleitung (54), bedruckten, Schutzhülle oder Verpackung (50) entnehmbar und nach Gebrauch, insbesondere nach Einmal-Gebrauch, in dieser Schutzhülle oder Verpackung (50) entsorgbar ist.

8. Vorrichtung gemäß mindestens einem der Ansprüche 1 bis 7, wobei, insbesondere an oder auf der zweiten Oberfläche (12u),
- mindestens ein Identitätsmodul (60),
- mindestens ein Temperaturerkennungsmodul (62),
- mindestens ein Speichermodul (64), insbesondere in Form mindestens einer Chipeinheit, und/oder
- mindestens ein Datenübertragungsmodul (66)
vorgesehen sind/ist.

9. Vorrichtung gemäß mindestens einem der Ansprüche 1 bis 8, wobei es sich bei der Körperflüssigkeit um Blut, um Fruchtwasser, um Speichel und/oder um Urin handelt.

10. Verfahren zum Messen mindestens einer Kenngröße oder mindestens eines Parameters mindestens einer Körperflüssigkeit mittels einer Vorrichtung (100) gemäß mindestens einem der Ansprüche 1 bis 9, wobei die durch das Pad (30s, 30r), insbesondere durch ein reaktives Modul (30r) des Pads (30s, 30r), bestimmbare Kenngröße und/oder Parameter durch mindestens ein Auswertemittel (40; 42) auswertbar ist.

## Claims

1. A device (100), in particular disposable device, for measuring at least one characteristic variable or at least one parameter of at least one body fluid, comprising
- at least one card-shaped carrier (10) having a first surface (12o) and having a second surface (12u) facing away from the first surface (12o),
- at least one, in particular needle-shaped, perforating, scribing or pricking means (20) on the first surface (12o), and
- at least one pad (30s, 30r) which is assigned to the perforating, scribing or pricking means (20) and by means of which the body fluid is separable and/or absorbable and the at least one characteristic variable or the at least one parameter is determinable,
**characterized in**
- **that** the pad (30s, 30r) is made of two parts, namely
-- in the form of a module (30s) separating the body fluid and assigned to the perforating, scribing or pricking means (20), and
-- in the form of a module (30r) reactive with the body fluid and assigned to the second surface (12u),
- **that** the characteristic variable which can be determined by the reactive module (30r) and/or the parameter which can be determined by the reactive module (30r) is evaluatable by at least one evaluation means (40; 42) which can be set in relation to the reactive module (30r), the evaluation means (40; 42) being given
-- by reference or comparison data related to the characteristic variable or to the parameter which are provided, for example printed, on the second surface (12u), and/or
-- by at least one external application or external facility related to the characteristic variable or to the parameter.

2. The device according to claim 1, wherein the perforating, scribing or pricking means (20), in particular together with the pad (30s, 30r), is at least partially retractable in the carrier (10).

3. The device according to claim 1 or 2, wherein the perforating, scribing or pricking means (20) comprises a spring mechanism, in particular for at least partially lowering the perforating, scribing or pricking means (20) in the carrier (10).

4. The device according to at least one of claims 1 to 3, wherein the perforating, scribing or pricking means (20) together with the pad (30s, 30r) are arranged in a recess or indentation (14) of the carrier (10).

5. The device according to at least one of claims 1 to 4, wherein the evaluation means (42) is a data processing program or an application or an app.

6. The device according to at least one of claims 1 to 5, wherein the perforating, scribing or pricking means (20) together with the pad (30s, 30r) is covered by a, in particular reclosable, cover (16), for example in the form of a protective film.

7. The device according to at least one of claims 1 to 6, wherein the device (100) is removable from a protective sheath or packaging (50), in particular provided with an opening perforation (52) and/or substantially transparent and/or printed, for example with an instruction manual (54), and is disposable after use, in particular after single use, in this protective sheath or packaging (50).

8. The device according to at least one of claims 1 to 7, wherein, in particular at or on the second surface (12u),
- at least one identity module (60),
- at least one temperature detection module (62),
- at least one memory module (64), in particular in the form of at least one chip unit, and/or
- at least one data transmission module (66)
is/are provided.

9. The device according to at least one of claims 1 to 8, wherein the body fluid is blood, amniotic fluid, saliva and/or urine.

10. Method for measuring at least one characteristic variable or at least one parameter of at least one body fluid by means of a device (100) according to at least one of claims 1 to 9, wherein the characteristic variable and/or parameter which can be determined by the pad (30s, 30r), in particular by a reactive module (30r) of the pad (30s, 30r), is evaluatable by at least one evaluation means (40; 42).

## Revendications

1. Dispositif (100), en particulier dispositif à usage unique, de mesure d'au moins une caractéristique ou d'au moins un paramètre d'au moins un fluide corporel, présentant
- au moins un support (10) en forme de carte avec une première surface (12o) et une seconde surface (12u) détournée de la première surface (12o),
- au moins un moyen de perforation, de fissure ou de piquage (20) en forme d'aiguille sur la première surface (12o) et
- au moins une plaquette (30s, 30r) attribuée au moyen de perforation, de fissure ou de piquage (20) au moyen duquel le fluide corporel peut être séparé et/ou reçu ainsi que l'au moins une caractéristique ou l'au moins un paramètre peut être déterminé(e),
**caractérisé en ce**
- **que** la plaquette (30s, 30r) est conçue en deux parties, à savoir
-- sous forme d'un module (30s) attribué au moyen de perforation, de fissure ou de piquage (20) séparant le fluide corporel et
-- sous forme d'un module (30r) attribué à la seconde surface (12u), réactif par rapport au fluide corporel,
- **que** la caractéristique pouvant être déterminée par le module réactif (30r) et/ou le paramètre pouvant être déterminé par le module réactif (30r) peut être évalué(e) par au moins un moyen d'évaluation (40 ; 42) pouvant être mis en relation avec le module réactif (30r), dans lequel le moyen d'évaluation (40 ; 42) est fourni
-- par des données de référence ou de comparaison liées à la caractéristique ou au paramètre, qui sont prévues, par exemple imprimées sur la seconde surface (12u), et/ou
-- par au moins une application externe ou un dispositif externe lié(e) à la caractéristique ou au paramètre.

2. Dispositif selon la revendication 1, dans lequel le moyen de perforation, de fissure ou de piquage (20), en particulier conjointement avec la plaquette (30s, 30r), peut être au moins partiellement enfoncé dans le support (10).

3. Dispositif selon la revendication 1 ou 2, dans lequel le moyen de perforation, de fissure ou de piquage (20) présente un mécanisme de ressort, en particulier pour enfoncer au moins partiellement le moyen de perforation, de fissure ou de piquage (20) dans le support (10).

4. Dispositif selon au moins l'une des revendications 1 à 3, dans lequel le moyen de perforation, de fissure ou de piquage (20) est disposé conjointement avec la plaquette (30s, 30r) dans un renfoncement ou une cavité (14) du support (10).

5. Dispositif selon au moins l'une des revendications 1 à 4, dans lequel le moyen d'évaluation (42) est un programme de traitement de données ou une application ou une appli.

6. Dispositif selon au moins l'une des revendications 1 à 5, dans lequel le moyen de perforation, de fissure ou de piquage (20), est couvert conjointement avec la plaquette (30s, 30r) par un couvercle (16) en particulier refermable, par exemple sous forme d'un film protecteur.

7. Dispositif selon au moins l'une des revendications 1 à 6, dans lequel le dispositif (100) peut être prélevé d'une enveloppe de protection ou d'un emballage (50) en particulier doté(e) d'une perforation d'ouverture (52) et/ou essentiellement transparente et/ou, par exemple imprimé(e) avec une notice d'utilisation (54), et selon les besoins, en particulier après une utilisation unique, peut être mis au rebut dans cette enveloppe de protection ou cet emballage (50).

8. Dispositif selon au moins l'une des revendications 1 à 7, dans lequel, en particulier contre ou sur la seconde surface (12u),
- au moins un module d'identité (60),
- au moins un module de détection de température (62),
- au moins un module de mémoire (64), en particulier sous forme d'au moins une unité de puce, et/ou
- au moins un module de transmission de données (66)
est/sont prévu(s).

9. Dispositif selon au moins l'une des revendications 1 à 8, dans lequel le fluide corporel est du sang, du liquide amniotique, de la salive et/ou de l'urine.

10. Procédé de mesure d'au moins une caractéristique ou d'au moins un paramètre d'au moins un fluide corporel au moyen d'un dispositif (100) selon au moins l'une des revendications 1 à 9, dans lequel la caractéristique et/ou le paramètre pouvant être déterminé(e) par la plaquette (30s, 30r), en particulier par un module réactif (30r) de la plaquette (30s, 30r), peut être évalué(e) par au moins un moyen d'évaluation (40 ; 42).
